# FASCICULE DE BREVET EUROPEEN

(11) **EP 0 966 268 B1**
(45) Date de publication et mention de la délivrance du brevet: **21.05.2003**
(21) Numéro de dépôt: 98937610.8
(22) Date de dépôt: 10.07.1998
(51) Int. Cl.: A61K 9/16, A61K 9/50, A61K 9/51

(54) **PARTICULES, EN PARTICULIER MICRO- OU NANOPARTICULES DE PROTEINES VEGETALES RETICULEES, LEUR PROCEDE DE PREPARATION ET COMPOSITIONS COSMETIQUES, PHARMACEUTIQUES OU ALIMENTAIRES EN CONTENANT**
PARTIKELN,INSBESONDERE MIKRO- ODER NANOPARTIKELN AUS QUERVERNETZTEN PFLANZLICHEN PROTEINEN,VERFAHREN ZU DEREN HERSTELLUNG UND DIESE ENTHALTENDE ARZNEIMITTEL ODER KOSMETIKA ODER NAHRUNGSMITTELZUBEREITUNGEN
CROSS-LINKED PLANT PROTEIN PARTICLES, IN PARTICULAR MICROPARTICLES OR NANOPARTICLES, PREPARATION METHOD AND COSMETIC, PHARMACEUTICAL OR FOOD COMPOSITIONS CONTAINING SAME

(30) Priorité: 15.07.1997 FR 9708968; 09.09.1997 US 944047
(43) Date de publication de la demande: 29.12.1999
(73) Titulaire: COLETICA, 69007 Lyon (FR)
(72) Inventeur: PERRIER, Eric, F-38138 Les Côtes d'Arey (FR); LEVY, Marie-Christine, F-51100 Reims (FR); LACAZETTE, Patricia, F-01390 Saint André de Corcy (FR); BUFFEVANT, Chantal, F-69390 Millery (FR)
(74) Mandataire: Portal, Gérard
(86) Numéro de dépôt international: FR9801502
(87) Numéro de publication internationale: WO99003450

(56) Documents cités:
- EP-A- 0 381 543
- WO-A-94/14420
- WO-A-95/33554
- GB-A- 2 040 863
- US-A- 4 780 321

## Description

La présente invention concerne essentiellement des particules, en particulier micro- ou nanoparticules de protéines végétales réticulées, leur procédé de préparation et compositions cosmétiques, pharmaceutiques ou alimentaires en contenant.

### ARRIERE-PLAN TECHNOLOGIQUE

Il est bien connu que l'encapsulation de substances actives offre des avantages importants tels que par exemple la protection de la substance ou sa libération lente ou différée au site d'utilisation.

Pour des applications dans les domaines cosmétiques, pharmaceutiques ou alimentaires, les matériaux les plus recherchés comme constituants de la paroi sont des substances naturelles, en particulier les protéines ou les polysaccharides, en raison de leur biocompatibilité.

Certains procédés de microencapsulation utilisant les protéines ou les polysaccharides découlent de la méthode de polycondensation interfaciale décrite par Chang (Chang T.M.S., Science, 1964, 146, 524-525). Selon cette méthode bien connue, on émulsionne une solution aqueuse d'une diamine dans une phase hydrophobe, puis on ajoute une solution d'un chlorure de diacide à l'émulsion. Les groupements aminés forment des liaisons amide avec le dichlorure d'acide à l'interface, ce qui donne une membrane individualisant des microcapsules. On sait que la réaction de polycondensation est favorisée par l'alcalinisation de la phase aqueuse car la réaction libère de l'acide chlorhydrique, lequel en l'absence d'agent alcalin, protone une partie des groupements aminés les rendant ainsi non acylables.
Il en est de même lorsque la diamine est remplacée par une protéine, pour former des microcapsules de protéine réticulée. Dans les procédés décrits dans la littérature antérieure, la solution aqueuse initiale de protéine est alcalinisée de manière à ce que tous les groupements aminés libres de la protéine soient sous forme non protonée, acylable. Ainsi par exemple, le document LEVY US-4,780,321 concerne la préparation de microcapsules à parois mixtes formées de polysaccharides et protéines réticulées. La solution aqueuse initiale de polysaccharide et protéine est alcaline, et de préférence d'un pH > 10. De même dans le document HUC 5,395,620, qui concerne des microcapsules à paroi d'atélocollagène et glycosaminoglycannes réticulés, le pH de la solution aqueuse initiale est de préférence basique, tous les exemples de ce brevet utilisant un tampon carbonate de pH 9,8.

Dans le document Mars FR-A-2-444 497, on utilise une solution aqueuse de protéine sans addition de substances alcalines ou tampons. Cependant, la phase aqueuse contient une forte concentration en protéine, au moins égale à 20% (p/p), de telle manière qu'une partie serve à neutraliser l'HCl formé (rôle de tampon), tandis que la fraction non protonée est utilisable pour la formation de la membrane.

Le glutaraldéhyde peut être également utilisé pour la fabrication de particules à partir de protéines. La réticulation est en général effectuée en milieu neutre ou alcalin. Ainsi par exemple la sérumalbumine peut être réticulée en solution à 20 % dans un tampon phosphate de pH 7,5 (Sheu M.T. et al., J. Parenter. Sci. Technol., 1986, 40, 253-258) avec 1% de glutaraldéhyde. Cependant le mécanisme de la réaction du glutaraldéhyde avec les protéines est complexe et reste mal élucidé. On sait que la réaction fait intervenir non seulement le glutaraldéhyde libre mais aussi des formes polymères provenant de la condensation du glutaraldéhyde sur lui-même, ces dérivés pouvant exister sous forme linéaire ou cyclique. La composition des solutions de glutaraldéhyde en ces différentes formes réactives est variable et dépend de divers facteurs, si bien que la nature des liaisons formées et le degré de réticulation ne sont pas complètement contrôlés, ce qui est un obstacle au développement industriel (Saleh A.M. et al., Int. J. Pharm., 1989, 57, 205-210). Par ailleurs, cet agent réticulant très réactif peut interférer avec divers principes actifs et diminuer ainsi leur biodisponibilité (Gupta P.K et Hung C.T., J. Microencapsulation, 1989, 6, 427-462). Enfin, des groupements aldéhyde libres peuvent être présents sur les particules (Magee G.A. et al., J. Controlled Release, 1995, 37, 11-19). La présence de tels groupements réactifs sur des particules destinées à l'utilisation humaine n'est pas souhaitable. Par exemple, des effets toxiques de particules vides ont été observés sur des macrophages (Suunders J . et al., Int. J. Pharm., 1991, 68, 265-270).

Les protéines citées dans les documents de la littérature antérieure sont des protéines animales, aucun ne mentionnant l'utilisation de protéines végétales.

Si l'on applique à des préparations de protéines végétales disponibles dans le commerce, les conditions décrites dans les documents antérieurs où la phase aqueuse utilisée pour dissoudre lesdites protéines végétales est un tampon au carbonate de sodium de pH = 9,8, il n'est pas possible d'obtenir des microcapsules stables. Les microcapsules sont obtenues en très faible quantité. Elles ont une membrane fragile : une partie d'entre elles apparaît souvent ouverte à l'examen microscopique. Elles forment de nombreux agrégats et se détériorent très rapidement en quelques jours à 45°C, à l'état de suspension aqueuse. Il en est de même lorsqu'on utilise pour dissoudre les protéines végétales des tampons phosphate de pH compris entre 7 et 8, ou simplement de l'eau distillée.

De même, on n'obtient pas de microcapsules stables si l'on utilise directement des préparations liquides contenant des protéines végétales telles que des laits de soja du commerce sans les tamponner, ou si on les tamponne par ajout de carbonate de sodium ou de phosphate de sodium.

### RESUME DE L'INVENTION

De manière parfaitement inattendue, il a été découvert que si l'on utilise, pour préparer les particules, des solutions de protéines végétales de pH proches de la neutralité ou même légèrement acides, c'est-à-dire dans un domaine de pH allant d'environ 4,5 à environ 8, et contenant au moins un sel d'acide carboxylique, en particulier un sel alcalin ou alcalino-terreux, de préférence un sel alcalin, on peut alors préparer des particules, en particulier des micro-ou nanoparticules stables de protéines végétales réticulées. De telles solutions de protéines végétales peuvent être obtenues, ou bien en utilisant des solutions tampons contenant lesdits sels, pour extraire les protéines contenues dans des préparations de protéines végétales pulvérulentes, ou bien en ajoutant lesdits sels à des préparations liquides contenant des protéines végétales telles que des laits de soja du commerce.

Ce résultat est d'autant plus inattendu que les pH de ces solutions sont peu élevés et que les concentrations en protéines dissoutes dans la phase aqueuse sont faibles, toujours inférieures à environ 5 % en poids.

### BUTS DE L'INVENTION

Ainsi, la présente invention a principalement pour objet de résoudre le nouveau problème technique consistant en la fourniture d'une solution permettant de préparer des particules, en particulier des micro- ou nanoparticules, des micro-ou nanocapsules, des micro- ou nanosphères, stables à partir de protéines végétales.

La présente invention a encore pour but principal de résoudre le nouveau problème technique consistant en la fourniture d'une solution permettant de préparer des particules, en particulier des micro- ou nanoparticules, des micro-ou nanocapsules, des micro- ou nanosphères, stables à partir de protéines végétales, tout en permettant éventuellement l'encapsulation d'une ou plusieurs substances actives à l'état de solution, de suspension ou d'émulsion.

La présente invention a encore pour but de résoudre les nouveaux problèmes techniques énoncés ci-dessus avec l'utilisation de procédés de fabrication simples, utilisables à l'échelle industrielle, en particulier dans l'industrie cosmétique, pharmaceutique, alimentaire. De préférence, cette solution doit permettre de préparer des particules ayant une taille de particule réglable à volonté, en particulier dans une plage de dimensions allant du nanomètre à quelques millimètres, en particulier d'environ 10 nanomètres à environ 3 mm.

### DESCRIPTION SUCCINTE DE L'INVENTION

Dans le cadre de l'invention, on entend par particule, une particule de forme essentiellement sphérique, qui peut avoir ou bien une structure vésiculaire, ou bien une structure homogène. Les particules comprennent donc toute sphère ou capsule. Pour les particules de structure vésiculaire, comportant un revêtement externe distinct formant une membrane autour du contenu, on parle généralement de capsules, et, en particulier de micro-ou de nano-capsules dans le cas d'une dimension de l'ordre du micromètre ou du nanomètre respectivement. Pour les particules de structure homogène, monolithique, on parle habituellement de sphères, et, en particulier de micro- ou de nano-sphères dans le cas d'une dimension de l'ordre du micromètre ou du nanomètre respectivement. L'ensemble de ces particules fait partie intégrante de la présente invention.

De préférence également, cette solution doit permettre de préparer des particules biocompatibles et biodégradables.

Ainsi, selon la présente invention, il a été découvert de manière parfaitement inattendue que l'on pouvait obtenir des particules stables en déclenchant une réaction de réticulation interfaciale entre des protéines végétales et un agent réticulant acylant polyfonctionnel en particulier un halogénure de diacide de préférence un chlorure de diacide, à l'interface des phases d'une émulsion, en particulier de type "eau-dans-huile" ou "huile-dans-eau".

Dans le cas d'une émulsion de type "eau-dans-l'huile", selon un premier mode de réalisation de l'invention, on émulsionne tout d'abord une phase aqueuse contenant les protéines végétales et au moins un sel d'acide carboxylique, dans une phase hydrophobe, puis on ajoute la solution d'agent réticulant à l'émulsion. On constate alors qu'il se forme à l'interface des gouttelettes aqueuses des membranes constituées de molécules réticulées de la protéine végétale.

Les particules sont suffisamment stables pour résister à une incubation prolongée à l'étuve à 45°C à l'état de suspension aqueuse sans destruction de leur structure.

Les particules sont rapidement lysées en présence d'une protéase, ce qui démontre leur biodégradabilité.

Selon les conditions choisies pour l'émulsification, la taille des particules peut varier de moins de 1 micron, c'est-à-dire être de taille nanométrique, par exemple en utilisant un homogénéiseur haute pression, à plusieurs centaines de micromètres ou même un ou plusieurs millimètres.

Il a été également découvert que l'on peut obtenir des particules, en particulier des capsules, de protéines végétales réticulées de grande taille, dont la taille moyenne dépasse 500 µm, une fraction de ces particules pouvant avoir un diamètre dépassant le millimètre. Il faut pour cela disperser la phase aqueuse contenant les protéines végétales et au moins un sel d'acide carboxylique, au sein d'une phase hydrophobe en utilisant des conditions d'agitation douce, éventuellement sans tensioactif, de manière à obtenir des gouttelettes dispersées de taille convenable. On procède ensuite à l'ajout de l'agent réticulant de manière à former la membrane de protéines réticulées.

Il a été également découvert que l'on peut augmenter le diamètre moyen des particules et la proportion de particules de taille supérieure au mm en incorporant à la phase aqueuse, ou à la phase hydrophobe, ou à l'une et l'autre phase, avant de procéder à l'émulsification, une petite quantité d'une substance insoluble et lipophile telle que par exemple de l'oxyde de titane, ou de l'oxyde de zinc, ou du talc, ou du stéarate de calcium, ou encore un colorant insoluble sous forme de pigment ou de laque.

Il a été également découvert que l'on pouvait obtenir des particules en déclenchant la réaction de réticulation au sein d'une émulsion de type "huile-dans-eau". Dans ce cas, on émulsionne une phase hydrophobe contenant un agent réticulant polyfonctionnel à au moins deux groupes acylants, de préférence un halogénure de diacide, en particulier un chlorure de diacide, au sein d'une phase aqueuse contenant des protéines végétales et au moins un sel d'acide carboxylique, et utilisée comme phase dispersante. On laisse la réaction se développer à l'interface et on maintient l'agitation pendant un temps convenable. On constate qu'il se forme une membrane autour des gouttelettes hydrophobes dispersées, donnant ainsi des particules à contenu hydrophobe, ici constituant en des capsules.

C'est ainsi qu'à partir de diverses préparations pulvérulentes de protéines végétales disponibles dans le commerce telles que des farines, des concentrats ou des isolats, on peut obtenir des solutions convenables en utilisant des tampons renfermant par exemple de l'acétate de sodium, ou du succinate de sodium, ou du citrate de sodium. Pour préparer les solutions de protéines végétales, on disperse un échantillon de la farine, ou du concentrat ou de l'isolat, dans la solution tampon, et on met en place une agitation, en s'aidant éventuellement d'un chauffage à température modérée, par exemple comprise entre 30 et 50°C. Après un temps convenable, généralement compris entre 5 min et 30 min, on élimine la fraction insoluble, par exemple par centrifugation. Le surnageant contenant les protéines végétales en solution dans le tampon, est alors utilisé comme phase aqueuse, pour préparer des particules par réticulation interfaciale au moyen d'agents réticulants, en particulier des halogénures de diacides.

De même, l'addition d'au moins un sel alcalin d'un acide carboxylique tel que l'acétate de sodium ou le citrate de sodium à des préparations liquides de protéines végétales telles que les laits de soja, donne une phase aqueuse permettant d'obtenir des particules stables à partir de ces laits.

Il a été également découvert que l'on pouvait obtenir des particules de protéines végétales réticulées de très petite taille, inférieure au micromètre, de taille intermédiaire, micrométrique, ou de grande taille, c'est-à-dire ayant un diamètre proche du millimètre ou même dépassant le millimètre et présentant une stabilité et une résistance mécanique élevées. Le diamètre moyen de ces particules, ici des microcapsules ou des capsules, peut dépasser 500 µm et même atteindre 800 à 900 µm, une fraction des particules ayant alors un diamètre dépassant le millimètre.

De telles particules de grande taille peuvent être obtenues par exemple en émulsionnant une phase aqueuse contenant les protéines végétales et au moins un sel alcalin d'un acide carboxylique, au sein d'une phase hydrophobe, dans des conditions compatibles avec l'obtention de gouttelettes dispersées de taille convenable, notamment en utilisant des conditions d'agitation douces, et éventuellement en supprimant l'addition d'un tensioactif. On obtient alors, de manière surprenante, des vésicules visibles à l'oeil nu, dont la membrane est élastique et résiste à la pression, et qui restent stables pendant des temps prolongés à la température de 45°C à l'état de suspension aqueuse.

De plus, il a été découvert que, dans des conditions données, y compris dans les conditions ci-dessus donnant des particules, microcapsules ou capsules, de diamètre moyen dépassant 500 µm, l'on peut augmenter le diamètre moyen des particules et la proportion des particules de diamètre supérieur à 1 mm, en incorporant à la phase aqueuse, ou à la phase hydrophobe, ou à l'une et l'autre phase, avant de procéder à l'émulsification, une petite quantité d'une substance insoluble et lipophile telle que par exemple de l'oxyde de titane, ou de l'oxyde de zinc, ou du stéarate de calcium, ou encore un colorant insoluble sous forme de pigment ou de laque. Lors du stade d'émulsification, la substance insoluble lipophile se place à l'interface huile/eau et stabilise ainsi les grosses gouttelettes, ce qui permet une réticulation interfaciale autour de ces grosses gouttelettes, lors de l'addition ultérieure de l'agent réticulant. Une membrane est ainsi formée donnant des particules de taille augmentée, ici en particulier des capsules et notamment des microcapsules ou des capsules ayant une dimension de l'ordre du millimètre pouvant même atteindre 2 mm ou 3 mm.

Les particules obtenues sont parfaitement visibles à l'oeil nu et, selon la nature de la substance insoluble lipophile utilisée, se présentent comme des vésicules de couleur blanche ou bien de la couleur de la substance colorée.

L'accès à des particules stables et solides d'une grande taille et éventuellement colorées représente un progrès technique important. Par exemple, le fait que les particules soient parfaitement visibles permet une vérification aisée de l'homogénéité des mélanges renfermant ces particules lorsqu'on les utilise à l'état de dispersion dans divers milieux. Les contrôles de stabilité des préparations qui les renferment sont également facilités.

### DESCRIPTION DETAILLEE DE L'INVENTION

Selon un premier aspect, la présente invention couvre des particules caractérisées en ce qu'elles comprennent au moins en surface une paroi formée de protéines végétales réticulées, en particulier au moyen d'une réticulation interfaciale entre les protéines végétales et un agent réticulant polyfonctionnel acylant, comprenant au moins deux groupes acylants, réalisant des liaisons covalentes entre les fonctions acylables des protéines et les groupements acyles de l'agent réticulant polyfonctionnel acylant.

Les fonctions acylables des protéines sont notamment les fonctions amine, les groupements hydroxyle, thiol, carboxylate.

Dans le cadre de l'invention, toute protéine végétale peut être utilisée sans limitation. De même, tout agent réticulant polyfonctionnel à au moins deux fonctions acylantes peut être utilisé sans limitation.

Selon un mode de réalisation avantageux de l'invention, les protéines végétales précitées sont extraites notamment de légumineuses, en particulier des plantes suivantes : lupin (genre Lupinus), soja (genre Glycine), pois (genre Pisum), pois chiche (Cicer), la luzerne (Medicago), fèverole (Vicia ), lentille (Lens), haricot (Phaseolus), du colza (Brassica), ou du tournesol (Helianthus), ou encore de céréales comme le blé, le maïs, l'orge, le malt, l'avoine.

Selon un autre mode de réalisation avantageux de l'invention, les protéines végétales précitées sont utilisées sous forme de préparations pulvérulentes telles que des farines, des concentrats, des isolats, ou de préparations liquides telles que des laits de soja.

Selon un autre mode de réalisation avantageux de l'invention, la solution aqueuse utilisée pour dissoudre les protéines végétales contenues dans les préparations pulvérulentes est une solution aqueuse tampon de pH compris entre environ 4,5 et environ 8.

Cette solution de pH compris entre environ 4,5 et environ 8 est de préférence obtenue avec un sel d'un acide carboxylique, en particulier un sel alcalin ou alcalino-terreux d'un acide carboxylique. De préférence, on utilise un sel alcalin d'un acide carboxylique, en particulier un sel de sodium ou de potassium, de préférence de sodium, à une concentration en poids comprise entre 0,1% et 20%.

L'acide carboxylique précité peut comporter un seul groupement carboxylique ou plusieurs de ces groupements. Les acides carboxyliques utilisables sont notamment les acide acétique, oxalique, malonique, succinique, glutarique, diméthylglutarique, adipique, fumarique, maléique, tartrique, malique, citrique, lactique, salicylique.

Selon un autre mode de réalisation avantageux de l'invention, la concentration de protéine végétale dans la phase aqueuse est comprise entre 0,5 et 5 % en poids.

Selon un autre mode de réalisation avantageux de l'invention, la quantité de sel d'acide carboxylique à ajouter aux préparations liquides de protéines végétales telles que le lait de soja est comprise entre 0,1 % et 20 %, mieux entre 5 et 15 %, mieux d'environ 10 % en poids.

Selon un autre mode de réalisation avantageux de l'invention, la substance insoluble lipophile à incorporer à la phase aqueuse ou à la phase hydrophobe, ou à l'une et l'autre phase, pour augmenter la taille des particules, est choisie de préférence parmi le groupe des sels insolubles d'acides gras et de métaux bivalents, comme le stéarate de calcium ou de magnésium, ou de talc, ou des oxydes métalliques tels que l'oxyde de titane, ou l'oxyde de zinc, ou des substances colorées insolubles sous forme de pigments tels que le DC Red 30, ou sous la forme de laques de calcium, d'aluminium, de baryum, ou de zirconium de divers colorants. Cette substance insoluble lipophile peut aussi être présente dans la masse des particules et/ou adsorbée à la surface de ces particules.

Comme exemples de sels insolubles d'acides gras utilisables, on citera les sels de calcium, magnésium, strontium, baryum d'acides carboxyliques à nombre de carbones égal ou supérieur à 12, tels que les acides laurique, myristique, palmitique, stéarique, oléique, linoléique.

Comme exemples de laques utilisables, on citera : l'indigocarmine aluminium lake (de couleur bleue), le Ponceau 4 R aluminium lake (rouge), le Sunset yellow FCF aluminium lake (orange).

Selon un autre mode de réalisation avantageux de l'invention, la quantité de ladite substance insoluble lipophile à incorporer à la phase aqueuse ou à la phase hydrophobe, ou à l'une et l'autre phase, pour augmenter la taille des particules, est comprise entre 0,01 % et 2 % en poids de la phase concernée.

Selon un autre mode de réalisation avantageux de l'invention, l'agent réticulant polyfonctionnel acylant précité est de préférence constitué par un dihalogénure d'acide ou un dianhydride d'acide. Comme dihalogénure d'acide celui-ci peut être choisi parmi le groupe consistant de dihalogénure de phtaloyle, de téréphtaloyle, de sébacoyle, de glutaryle, d'adipoyle ou de succinyle. On préfère utiliser un dichlorure de ces acides.

Selon un second aspect, la présente invention couvre aussi l'utilisation de ces particules pour la fabrication d'une composition cosmétique, pharmaceutique et notamment dermatologique, ou alimentaire.

Selon un troisième aspect, la présente invention couvre encore une composition cosmétique, pharmaceutique, notamment dermatologique, ou alimentaire contenant de telles particules.

Dans ces compositions, la proportion d'incorporation des particules de l'invention pourra varier dans de larges limites et sera, de préférence, comprise entre 0,01 et 10 % en poids par rapport au poids total de la composition finale.

Selon un quatrième aspect, la présente invention couvre encore un procédé de fabrication de particules précitées à paroi formée de protéines végétales réticulées, caractérisé en ce qu'il comprend :
a) la formation d'une solution aqueuse de pH compris entre environ 4,5 et environ 8 contenant en solution au moins une protéine végétale, et au moins un sel d'acide carboxylique ;
b) la formation d'une phase huileuse ;
c) la formation d'une émulsion par mélange sous agitation de la phase aqueuse et de la phase huileuse ci-dessus ;
d) la réticulation interfaciale de ladite protéine végétale à l'aide d'un agent réticulant polyfonctionnel acylant à au moins deux groupes acylants pendant une période de temps suffisante pour obtenir des particules comprenant au moins en surface des parois formées de protéines végétales réticulées par ledit agent réticulant ; et
e) si désiré, la séparation des particules ainsi préparées.

Selon un autre mode de réalisation avantageux du procédé de l'invention, la solution aqueuse précitée est préparée à partir de préparations pulvérulentes de protéines végétales, en utilisant un tampon de pH compris entre environ 4,5 et environ 8 contenant un sel d'acide carboxylique à une concentration en poids généralement comprise entre 0,1 et 20 %. Les sels d'acides carboxyliques utilisés pour fabriquer la solution aqueuse tampon ont été décrits précédemment.

Comme tampon actuellement préféré, on pourra utiliser un tampon obtenu à l'aide d'un acide carboxylique, de préférence biocompatible, par exemple choisi parmi le groupe consistant d'un acétate, d'un succinate, d'un citrate.

Selon un mode de réalisation avantageux de l'invention, la solution aqueuse précitée est obtenue à partir d'une préparation liquide de protéines végétales par ajout d'un sel d'acide carboxylique à une concentration en poids comprise entre 0,1% et 20%.

Selon un mode de réalisation avantageux de l'invention, l'agent réticulant polyfonctionnel précité est choisi parmi un dihalogénure d'acide ou un dianhydride d'acide. Comme indiqué précédemment, le dihalogénure est de préférence un dichlorure et on utilisera de préférence un acide parmi la liste précédemment indiquée.

Selon un mode de réalisation avantageux de l'invention, le rapport du poids d'agent réticulant au poids de protéine utilisé est compris entre 0,03 et 70 parties en poids d'agent réticulant pour une partie en poids de protéine. La réaction de réticulation interfaciale est avantageusement réalisée à une température comprise entre 4 et 80°C, de préférence entre 15 et 30°C, et à pression atmosphérique .

Selon un mode de réalisation avantageux du procédé de l'invention, on utilisera pour former la phase huileuse, de préférence soit un solvant organique qui peut être facilement éliminé tel que le cyclohexane ou un mélange chloroforme/cyclohexane en particulier à un rapport 1:4 v/v, soit de préférence une huile biocompatible, en particulier une huile végétale ou minérale, ou un ester, ou un mélange d'esters d'acide gras, en particulier une huile de coco, le cocoate d'éthyle-2-hexyle.

Selon un mode de réalisation particulier de l'invention, on réalisera un procédé en émulsion de type eau-dans-huile.

Selon un autre mode de réalisation, on réalisera un procédé en émulsion de type huile-dans-eau.

Dans le cadre de l'un quelconque des procédés de l'invention, on pourra utiliser avantageusement un agent tensioactif pour faciliter ou stabiliser l'émulsion, choisi parmi un agent tensioactif bien connu à l'homme de l'art. On utilisera, dans le cadre de l'invention, de préférence un tensioactif de type ester de sorbitol tel que le sorbitan trioléate commercialisé sous le nom commercial de Span 85® de la société ICI.

D'autre part, dans le cadre de l'invention, on pourra utiliser un additif insoluble tel qu'un pigment pour obtenir de grandes particules, en particulier des capsules c'est-à-dire ayant une taille au moins égale à environ 1 mm ou même 2 ou 3 mm. A titre d'additif particulièrement intéressant dans le cadre de l'invention, on peut citer, sans limitation, des oxydes métalliques tels que l'oxyde de titane, de l'oxyde de zinc, du talc, des substances colorées insolubles sous forme de pigments ou de laques.

Dans le cadre de l'invention, on peut ainsi obtenir des particules ayant une dimension réglable à volonté depuis les plus petites tailles jusqu'aux grandes tailles, c'est-à-dire de taille nanométrique jusqu'à de grandes tailles supérieures à 1 mm, c'est-à-dire pouvant aller jusqu'à environ 2 mm ou même 3 mm. L'invention inclut aussi dans la définition des "particules", des capsules ou des sphères, donc notamment des nanocapsules ou nanosphères et des microcapsules ou des microsphères.

Egalement, les particules obtenues dans le cadre de l'invention, en particulier les microcapsules, nanocapsules ou capsules, peuvent être indifféremment à contenu aqueux ou huileux, et présentent un aspect satisfaisant. Elles sont solides, faciles à disperser dans divers milieux hydrophiles ou lipophiles. Les particules selon l'invention sont stables à 45°C, à l'état de suspension aqueuse, qu'elles soient à contenu aqueux ou à contenu huileux.

On peut également incorporer dans le cas des procédés de l'invention diverses substances en suspension, par exemple des pigments, ou en solution, comme par exemple un sucre tel que le glucose, ou en émulsion, par exemple telle qu'une huile, en particulier une huile de paraffine.

Les particules selon l'invention sont également biodégradables car elles peuvent être lysées rapidement par une enzyme telle que la trypsine ou autre enzyme bien connue à l'homme de l'art.

Pour la fabrication de nanoparticules, nanosphères ou nanocapsules, on pourra utiliser le procédé décrit dans la demande antérieure du déposant FR-A-2 683 159 = WO 93/08908.

On comprend que l'invention permet de réaliser des particules, en particulier des sphères ou des capsules, telles que nanosphères ou nanocapsules, microsphères ou microcapsules, qui permettent d'encapsuler des substances, en particulier des principes actifs, dont les principes actifs lipophiles tels que huile végétale, minérale, ou synthétique, des dérivés de vitamine A et de vitamine E, etc., ainsi que des principes actifs hydrophiles tels que des extraits végétaux, l'acide ascorbique ou vitamine C, PMG, glucose, des pigments organiques et pigments minéraux.

La stabilité de ces particules sous différentes conditions a pu être observée à des températures allant de 4°C à 90°C, pour des pH allant de 3 à 11. Ainsi une stabilité de durée supérieure à 6 mois à 45°C, a pu être observée, que ce soit en milieu sec ou hydraté.

La biodégrabilité des particules a été démontrée à l'aide de différentes enzymes, ces enzymes pouvant être la trypsine, la chymotrypsine ou une autre protéase.

Ces particules sont également parfaitement biocompatibles et ne procurent aucune irritation cutanée, oculaire, aucune toxicité orale.

Les particules selon l'invention peuvent être utilisées, quelle que soit leur taille, dans des compositions cosmétiques pour réaliser des formulations de type émulsion eau-dans-huile ou huile-dans-eau, ou des gels hydrophiles, des gels hydrophobes, des shampooings et des gels douche.

Ainsi, comme il a été dit précédemment, ces particules ou capsules peuvent être utilisées pour préparer des compositions cosmétiques, pharmaceutiques, notamment dermatologiques, ou alimentaires en les combinant à divers ingrédients actifs ou excipients bien connus de l'homme de l'art.

D'autres buts, caractéristiques et avantages de l'invention apparaîtront clairement à l'aide des exemples suivants donnés simplement à titre d'illustration et qui ne sauraient donc en aucune façon limiter la portée de l'invention. Les exemples font partie intégrante de l'invention. Ainsi, toute caractéristique qui apparaît être nouvelle par rapport à un état de la technique quelconque fait partie intégrante de l'invention dans sa fonction et sa caractéristique générale. Dans les exemples, les pourcentages sont donnés en poids, sauf indication contraire. En outre, la température est la température ambiante ou bien est exprimée en degré celsius, sauf indication contraire. La pression est la pression atmosphérique, sauf indication contraire.

### Exemple 1 de l'invention : Préparation de microcapsules à paroi formée de protéines de lupin réticulées

### a) Préparation de la phase aqueuse :

On disperse 0,75 g de farine de lupin (*Farine ultra-fine de lupin blanc doux* (CANA) à 45% de protéines) dans 15 ml de tampon acétate pH 7,4. On agite sous agitation magnétique pendant 10 min, puis on centrifuge et on sépare le surnageant.

### b) Emulsification

On disperse 6 ml du surnageant dans 30 ml de cyclohexane à 2% de sorbitan trioleate (Span 85®) par agitation de 5 min à 2000 rpm.

### c) Réticulation

On ajoute 40 ml d'une solution de chlorure de téréphtaloyle à 5% (p/v) dans un mélange de chloroforme : cyclohexane (1:4 v/v). Après agitation de 30 min, les microcapsules sont séparées par centrifugation puis lavées par remise en suspension dans du cyclohexane, puis dans l'alcool additionné de 2% de polysorbate, dans l'alcool à 95%, puis dans l'eau.

On obtient un sédiment blanc. L'examen microscopique montre de belles microcapsules sphériques, transparentes, très légèrement piquetées, à membrane nette et régulière, solides, de taille comprise entre 20 et 50 µm.

Les microcapsules sont intactes après 5 mois de conservation à l'étuve à 45°C à l'état de suspension aqueuse.

### Exemple 2 de l'invention : Microcapsules à paroi formée de protéines de lupin réticulées

Le protocole de l'exemple 1 est reproduit en remplaçant le tampon acétate de pH 7,4 par un tampon acétate de pH 6,8. On obtient également un sédiment blanc formé de belles microcapsules sphériques, solides, de taille comprise entre 20 et 60 µm. Les microcapsules sont intactes après 16 semaines de conservation à l'étuve à 45°C à l'état de suspension aqueuse.

### Exemple 3 de l'invention : Microcapsules à paroi formée de protéines de lupin réticulées

Le protocole de l'exemple 1 est reproduit en remplaçant le tampon acétate de pH 7,4 par un tampon acétate de pH 5,9. On obtient également un sédiment blanc formé de belles microcapsules sphériques, solides, de taille comprise entre 20 et 60µm. Les microcapsules sont intactes après 16 semaines de conservation à l'étuve à 45°C à l'état de suspension aqueuse.

### Exemple 4 de l'invention : Microcapsules à paroi formée de protéines de lupin réticulées

Le protocole de l'exemple 1 est reproduit en remplaçant le tampon acétate de pH 7,4 par un tampon succinate pH 6. On obtient de belles microcapsules sphériques, solides, de taille comprise entre 10 et 50 µm. Les microcapsules sont intactes après 16 semaines de conservation à l'étuve à 45°C à l'état de suspension aqueuse.

### Exemple 5 de l'invention : Microcapsules à paroi formée de protéines de lupin réticulées

Le protocole de l'exemple 1 est reproduit en remplaçant le tampon acétate de pH 7,4 par un tampon citrate pH 6. On obtient de belles microcapsules sphériques, solides, de taille comprise entre 20 et 60 µm. Les microcapsules sont intactes après 15 semaines de conservation à l'étuve à 45°C à l'état de suspension aqueuse.

### Exemple 6 de l'invention : Microcapsules à paroi formée de protéines de pois réticulées

Le protocole de l'exemple 1 est reproduit en utilisant, au lieu de farine de lupin, un isolat de protéines de Pois (à 90% de protéines : Pisane HD®, COSUCRA). On obtient de belles microcapsules sphériques, solides, de taille comprise entre 20 et 70 µm. Les microcapsules sont intactes après 4 semaines de conservation à l'étuve à 45°C à l'état de suspension aqueuse.

### Exemple 7 de l'invention : Microcapsules à paroi formée de protéines de féverole réticulées

Le protocole de l'exemple 1 est reproduit en utilisant, au lieu de farine de lupin, un concentrat de protéines de Féverole (à 50% de protéines : Concentrat 50®, Gemef Industrie). On obtient de belles microcapsules sphériques, solides, de taille comprise entre 20 et 60 µm. Les microcapsules sont intactes après 4 mois de conservation à l'étuve à 45°C à l'état de suspension aqueuse.

### Exemple 8 de l'invention : Microcapsules à paroi formée de protéines de lupin réticulées

### a) Préparation de la phase aqueuse :

On disperse 0,75 g de farine de lupin (*Farine ultra-fine de lupin blanc doux* (CANA) à 45% de protéines) dans 15 ml de tampon acétate pH 7,4, porté à la température de 35°C. On agite sous agitation magnétique pendant 15 min, puis on centrifuge et on sépare le surnageant.

### b) Emulsification

On disperse 6 ml du surnageant dans 30 ml de cocoate d'éthyl 2 hexyle à 2% de sorbitan trioleate (Span 85®) par agitation de 5 min à 2000 rpm.

### c) Réticulation

On procède pour la réticulation comme décrit à l'exemple 1.

On obtient des microcapsules de taille comprise entre 20 et 60 µm, parfaitement sphériques.

Elles résistent plus de 14 semaines à une température de 45°C, à l'état de suspension aqueuse.
*Essai de lyse dans la trypsine* : 250 mg de microcapsules humides sont introduits dans un tube contenant 7,5 ml d'une solution à 0,4 % de trypsine (type II-S, from porcine pancreas, Sigma) dans un tampon de pH 7,5. Le tube est incubé à 37°C et une agitation magnétique est installée. La lyse est suivie par examen microscopique jusqu'à disparition complète des microcapsules.
Résultat : les microcapsules ont complètement disparu après 20 min.

### Exemple 9 de l'invention : Microcapsules à paroi formée de protéines de lupin réticulées contenant un principe actif hydrosoluble.

### a) Préparation de la phase aqueuse :

On disperse 0,75 g de farine de lupin (*Farine ultra-fine de lupin blanc doux* (CANA) à 45% de protéines) dans 15 ml de tampon succinate pH 6 porté à la température de 35°C. On installe une agitation magnétique de 15 min, puis on centrifuge et on sépare le surnageant. On dissout du glucose dans le surnageant à la concentration de 3%.

On opère ensuite pour l'émulsification et la réticulation comme décrit à l'exemple 8.

Les microcapsules sont séparées par centrifugation puis lavées plusieurs fois avec le cocoate d'éthyl hexyle. On obtient des microcapsules parfaitement formées, de taille comprise entre 20 et 70 µm, contenant du glucose.

### Exemple 10 de l'invention : Microcapsules à paroi formée de protéines de pois réticulées

Le protocole décrit à l'exemple 8 est reproduit en remplaçant la farine de lupin par un isolat de protéines de Pois (à 90% de protéines : Pisane HD®, Cosucra), et en remplaçant le tampon acétate pH 7,4 par un tampon succinate de pH 6.

On obtient des microcapsules sphériques de taille comprise entre 10 et 60 µm, qui résistent plus de 8 semaines à une température de 45°C, à l'état de suspension aqueuse. Ces microcapsules sont lysées dans la trypsine, dans les conditions décrites à l'exemple 8, en 8 min.

### Exemple 11 de l'invention : Microcapsules à paroi formée de protéines de féverole réticulées

Le protocole décrit à l'exemple 8 est reproduit en remplaçant la farine de lupin par un concentrat de protéines de Féverole (à 50% de protéines : Concentrat 50®, Gemef Industrie). On obtient des microcapsules sphériques de taille comprise entre 10 et 50 µm, qui résistent plus de 8 semaines à une température de 45°C, à l'état de suspension aqueuse. Les microcapsules sont lysées dans la trypsine, dans les conditions décrites à l'exemple 8, en 75 min.

### Exemple 12 de l'invention : Microcapsules de protéines de féverole réticulées, de grande taille

Le protocole décrit à l'exemple 11 est reproduit en remplaçant le tampon acétate par le tampon citrate pH 6, en supprimant l'addition de trioléate de sorbitane et en diminuant la vitesse d'agitation à 600 rpm. On obtient un volumineux sédiment de capsules opaques, visibles à l'oeil nu, et sédimentant rapidement.

L'analyse de taille de 300 capsules, effectuée au microscope avec un oculaire muni d'une échelle micrométrique, donne une marge de taille comprise entre 217 et 1643 µm, avec un diamètre moyen de 735 µm, et une proportion de 18% de microcapsules de taille supérieure au mm.

On observe que la membrane des microcapsules est élastique et solide: si on exerce une pression sur la lamelle d'observation microscopique, le diamètre des microcapsules augmente pendant que s'exerce la pression, pour reprendre sa valeur initiale lorsque la pression cesse.

Les microcapsules sont intactes après 7 semaines de conservation à l'étuve à 45°C à l'état de suspension aqueuse.

### Exemple 13 de l'invention : Microcapsules de protéines de féverole réticulées : augmentation de la taille par addition d'oxyde de titane à la phase hydrophobe.

Le protocole décrit à l'exemple 12 est reproduit en dispersant 0,1% d'oxyde de titane dans la phase huileuse, avant l'émulsification. On obtient des microcapsules à membrane solide et élastique, sédimentant rapidement.

L'analyse de taille, effectuée comme à l'exemple 12 donne une marge de taille de 217 à 2170 µm, avec un diamètre moyen de 899 µm et une proportion de 43 % de microcapsules de taille supérieure au mm.

Cet exemple démontre que l'addition d'oxyde de titane à la phase huileuse permet d'augmenter de manière importante le diamètre moyen des microcapsules et la proportion de microcapsules de taille supérieure au mm.

### Exemple 14 de l'invention : Microcapsules de protéines de lupin réticulées : augmentation de la taille par addition de colorant Red DC 30 à la phase aqueuse.

Le protocole décrit à l'exemple 8 est reproduit, en remplaçant le tampon acétate par le tampon succinate pH 6, en dispersant dans la phase aqueuse 0,1% du pigment rouge Red DC 30, en supprimant le trioléate de sorbitane, et en diminuant la vitesse d'agitation à 600 rpm. On obtient un sédiment formé de vésicules de couleur rouge, sphériques. On observe que la membrane est élastique et solide : si on exerce une pression sur la lamelle d'observation microscopique, le diamètre des microcapsules augmente pendant que s'exerce la pression, pour reprendre sa valeur initiale lorsque la pression cesse.

Les microcapsules sont intactes après 3 mois de conservation à l'étuve à 45°C à l'état de suspension aqueuse. Ces microcapsules sont lysées par la trypsine en 12 min, dans les conditions décrites à l'exemple 8.

L'analyse de taille, effectuée comme à l'exemple 12, donne une marge de taille de 186 à 1240 µm, un diamètre moyen de 632 µm, et une proportion de 6% de microcapsules de taille supérieure au mm.

Un essai comparatif effectué dans des conditions identiques mais en omettant l'addition du pigment rouge Red DC 30, donne des microcapsules de diamètre très nettement inférieur, avec une marge de taille de 150 à 800 µm, un diamètre moyen de 322 µm, et aucune capsule de diamètre supérieur au mm .

### Exemple 15 de l'invention : Microcapsules de protéines de lupin réticulées de grande taille obtenues avec addition de laque d'indigocarmin à la phase aqueuse.

Le protocole décrit à l'exemple 14 est reproduit en remplaçant le pigment Red DC 30 par la laque : indigocarmine aluminium lake (Colorcon). On obtient des microcapsules colorées en bleu à membrane solide et élastique. Les microcapsules sont intactes après 5 semaines de conservation à l'étuve à 45°C à l'état de suspension aqueuse.

L'analyse de taille donne une marge de taille de 217 à 1023 µm, un diamètre moyen de 536 µm, et une proportion de 0,3 % de microcapsules de taille supérieure au mm.

### Exemple N°16 : Microcapsules de protéines de lupin réticulées de grande taille, obtenues avec addition de stéarate de calcium à la phase aqueuse

Le protocole décrit à l'exemple 14 est reproduit en remplaçant le pigment Red DC 30 par du stéarate de calcium. On obtient des microcapsules blanches qui restent intactes après 3 semaines de conservation à l'étuve à 45°C à l'état de suspension aqueuse.

L'analyse de taille donne une marge de taille de 279 à 1240 µm, un diamètre moyen de 679 µm, et une proportion de 7 % de microcapsules de taille supérieure au mm.

### Exemple 17 de l'invention : Microcapsules de protéines de lupin réticulées de grande taille obtenues avec addition de talc à la phase aqueuse

Le protocole décrit à l'exemple 14 est reproduit en remplaçant le pigment Red DC 30 par du talc. On obtient des microcapsules blanches qui restent intactes après 7 semaines de conservation à l'étuve à 45°C à l'état de suspension aqueuse.

L'analyse de taille donne une marge de taille de 217 à 1147 µm, un diamètre moyen de 666 µm, et une proportion de 5 % de microcapsules de taille supérieure au mm.

### Exemple 18 de l'invention : Microcapsules de protéines de lupin réticulées de grande taille obtenues avec addition d'oxyde de titane à la phase aqueuse.

Le protocole décrit à l'exemple 14 est reproduit en remplaçant le pigment Red DC 30 par de l'oxyde de titane. On obtient des microcapsules blanches. L'analyse de taille, effectuée comme à l'exemple 12, donne une marge de taille de 248 à 1271 µm, un diamètre moyen de 620 µm, et une proportion de 2 % de microcapsules de taille supérieure au mm.

### Exemple 19 de l'invention : Microcapsules de protéines de lupin réticulées de grande taille obtenues avec addition d'oxyde de titane à la phase hydrophobe

Le protocole décrit à l'exemple 18 est reproduit, en incorporant l'oxyde de titane, non pas à la phase aqueuse mais à la phase hydrophobe, et en l'utilisant à la concentration de 0,05%. On obtient des microcapsules blanches. L'analyse de taille donne une marge de taille de 155 à 1674 µm, un diamètre moyen de 679 µm, et une proportion de 17 % de microcapsules de taille supérieure au mm.

### Exemple N°20 : Microcapsules de protéines de lupin réticulées de grande taille obtenues avec addition d'oxyde de titane à la phase hydrophobe

Le protocole décrit à l'exemple 19 est reproduit, en utilisant l'oxyde de titane à la concentration de 0,1%. On obtient des microcapsules blanches. L'analyse de taille donne une marge de taille de 217 à 1860 µm, un diamètre moyen de 852 µm, et une proportion de 37% de microcapsules de taille supérieure au mm.

### Exemple 21 de l'invention : Microcapsules de protéines de lupin réticulées de grande taille obtenues avec addition d'oxyde de titane à la phase aqueuse et à la phase hydrophobe

Le protocole décrit à l'exemple 20 est reproduit, en incorporant l'oxyde de titane à la concentration de 0,05% à la phase aqueuse, et 0,05% à la phase hydrophobe.

On obtient des microcapsules blanches. L'analyse de taille donne une marge de taille de 248 à 1798 µm, un diamètre moyen de 825 µm, et une proportion de 26 % de microcapsules de taille supérieure au mm.

### Exemple 22 de l'invention : Microcapsules de protéines de pois réticulées de grande taille obtenues avec addition d'oxyde de titane à la phase hydrophobe

Le protocole décrit à l'exemple 10 est reproduit, en supprimant le trioléate de sorbitane, en diminuant la vitesse d'agitation à 1000 rpm, et en incorporant de l'oxyde de titane à la phase hydrophobe à la concentration de 0,1%. On obtient des microcapsules blanches. L'analyse de taille donne une marge de taille de 124 à 1147 µm, un diamètre moyen de 565 µm, et une proportion de 6 % de microcapsules de taille supérieure au mm.

Un essai comparatif effectué dans des conditions identiques mais en omettant l'addition de l'oxyde de titane donne des microcapsules de diamètre très nettement inférieur, avec une marge de taille de 124 à 868 µm, un diamètre moyen de 345 µm, et aucune capsule de diamètre supérieur au mm.

### Exemple 23 de l'invention : Microcapsules de protéines de lupin réticulées à contenu huileux.

### - Préparation de la phase aqueuse :

On disperse 2 g de farine de lupin dans 40 ml de tampon succinate pH 6, porté à la température de 35°C. On agite sous agitation magnétique pendant 15 min, puis on centrifuge et on sépare le surnageant.

### - Préparation de la phase huileuse:

On ajoute à 6 ml d'huile de paraffine fluide, 0,3 ml de chlorure de sébacoyle.

### - Emulsification-réticulation:

On disperse la phase huileuse dans 30 ml de la phase aqueuse par agitation à 5000 rpm, et on laisse se développer la réaction de réticulation pendant 60 min. Les microcapsules sont ensuite lavées plusieurs fois à l'eau distillée.

On obtient un sumageant formé de microcapsules bien formées, sphériques, de taille 10-150 µm. Toute l'huile est encapsulée. Les microcapsules sont intactes après 3 mois de conservation à l'étuve à 45°C à l'état de suspension aqueuse.

### Exemple 24 de l'invention : Microcapsules de protéines de féverole réticulées à contenu huileux

Le protocole décrit à l'exemple 23 est appliqué, en remplaçant la farine de lupin par la préparation de protéines de féverole : Concentrat 50®, et en utilisant le tampon acétate de pH 7,4 au lieu du tampon succinate de pH 6. On obtient un surnageant formé de microcapsules bien formées, de taille 30-150 µm. Toute l'huile est encapsulée.

Les microcapsules sont intactes après 2 mois de conservation à l'étuve à 45°C à l'état de suspension aqueuse.

### Exemple 25 de l'invention : Microcapsules préparées à partir de lait de soja

### a) Préparation de la phase aqueuse :

On dissout 1 g d'acétate de sodium trihydrate dans 1 ml d'eau distillée et on mélange cette solution avec 10 ml de lait de soja Bjorg® (Distriborg).

### b) Emulsification

On disperse 6 ml de phase aqueuse dans 30 ml de cyclohexane à 2% de sorbitan trioléate, par agitation de 5 min à 2000 rpm.

### c) Réticulation

On ajoute 40 ml d'une solution de chlorure de téréphtaloyle à 5% (p/v) dans un mélange de chloroforme : cyclohexane (1:4 v/v). Après agitation de 30 min, les microcapsules sont séparées par centrifugation puis lavées comme décrit à l'exemple 1.

On obtient après centrifugation un important sédiment blanc crème. L'examen microscopique montre de belles microcapsules sphériques, à contenu finement piqueté, à membrane nette, de taille : 10-70 µm. Incubées à l'étuve à 45°C, les microcapsules sont intactes après un an de conservation à l'état de suspension aqueuse, après 8 mois et demi à l'état de suspension dans un gel de carbopol ou de xanthane, ou dans des huiles de silicone ou d'arachide gélifiées, ou dans une microémulsion lipidique gélifiée.

Des études toxicologiques ont été réalisées et font l'objet de l'exemple 43.

### Exemple 26 de l'invention : Préparation de microcapsules à partir de lait de soja

Le protocole décrit à l'exemple 25 est appliqué en remplaçant le lait de soja BJORG par du lait de soja CEREAL®. On obtient des microcapsules sphériques de taille 10-60 µm, intactes après 5 mois et demi de séjour à l'étuve à *45°C,* à l'état de suspension aqueuse.

### Exemple 27 de l'invention : Préparation de microcapsules à partir de lait de soja

Le protocole décrit à l'exemple 25 est appliqué en remplaçant le lait de soja BJORG par du lait de soja GAYLORD HAUSER®. On obtient des microcapsules sphériques de taille 10-70 µm, intactes après 5 mois et demi de séjour à l'étuve à 45°C, à l'état de suspension aqueuse.

### Exemple 28 de l'invention : Préparation de microcapsules à partir de lait de soja

Le protocole décrit à l'exemple 25 est reproduit en remplaçant l'acétate de sodium par du citrate de sodium dihydrate ajouté à raison de 0,65g pour 1 ml d'eau, en dispersant dans la phase aqueuse préalablement portée à la température de 35°C, 0,1% d'oxyde de titane, en remplaçant le cyclohexane par du cocoate d'éthyl 2 hexyle, en supprimant l'addition de sorbitan trioléate, et en abaissant la vitesse d'agitation à 600 rpm.

On obtient un volumineux sédiment blanc formé de belles microcapsules à membrane solide et élastique, de taille 100 à 900 µm.

### Exemple 29 de l'invention : Microcapsules préparées à partir de lait de soja et contenant une émulsion d'huile

### a) Préparation de la phase aqueuse :

On dissout 2 g d'acétate de sodium trihydrate dans 2ml d'eau distillée et on mélange cette solution avec 20 ml de lait de soja Bjorg®.

### b) Première émulsification : Huile/Eau

On disperse 5 ml d'huile de paraffine fluide dans 15 ml de la phase aqueuse précédente par agitation de 5 min à 5000 rpm.

### c) Deuxième émulsification : Eau/Huile

On prélève 6 ml de l'émulsion précédente et on les disperse dans 30 ml de cocoate d'éthyl 2 hexyle à 1 % de sorbitan trioleate par agitation de 5 min à 1200 rpm.

### d) Réticulation

On ajoute 40 ml d'une solution de chlorure de téréphtaloyle à 5% (p/v) dans le cocoate. Après agitation de 30 min, les microcapsules sont séparées par centrifugation puis lavées comme décrit à l'exemple 1.

On obtient des microcapsules de taille 10-100 µm renfermant des gouttelettes réfringentes d'huile de paraffine.

### Exemple 30 de l'invention : Préparation de microcapsules à contenu huileux à partir de lait de soja

### a) Préparation de la phase aqueuse :

On dissout 5 g d'acétate de sodium trihydrate dans 5 ml d'eau distillée et on mélange cette solution avec 50 ml de lait de soja Bjorg®.

### b) Préparation de la phase huileuse :

On ajoute à 12 ml d'huile de paraffine fluide, 0,6 ml de chlorure de sébacoyle.

### c) Emulsification-réticulation :

On disperse la phase huileuse dans la phase aqueuse par agitation à 5000 rpm, et on laisse se développer la réaction de réticulation pendant 60 min. Les microcapsules sont ensuite lavées plusieurs fois à l'eau distillée.

On obtient un surnageant de couleur crème formé de microcapsules bien formées, sphériques, de taille 100-200 µm. Toute l'huile est encapsulée.

Incubées à l'étuve à 45°C, les microcapsules sont intactes après 8 mois de conservation à l'état de suspension aqueuse, après 2 mois et demi à l'état de suspension dans des huiles de silicone ou d'arachide gélifiées.

### Exemple 31 de l'invention : Préparation de nanocapsules à paroi en protéines végétales

### a) Fabrication de la solution de protéines végétales nécessaire à la fabrication des nanocapsules

200 g de farine de lupin sont dispersés dans 4 1 de tampon succinate pH 6 porté à température de 35°C. Après agitation magnétique pendant 15 min, la solution est centrifugée et le surnageant récupéré. Il est utilisé pour les étapes ultérieures.

### b) Préparation de la phase huileuse

30 ml de chlorure de sébacoyle sont ajoutés à 100 ml d'huile de paraffine fluide. Le mélange est effectué à température ambiante et la solution homogène est utilisée pour les étapes ultérieures.

### c) Emulsification/réticulation

Les solutions préparées en a) et b) sont additionnées en continu et envoyées dans un homogénéisateur haute pression à une pression d'homogénéisation comprise entre 300 et 1 200 bars, par exemple 800 bars. Les nanocapsules obtenues sont de tailles inférieures à 1 micron, sont remarquablement stables et sont utilisables dans un grand nombre de formulations cosmétiques, y compris les formulations cosmétiques hydratées, sans problème de dégradation au cours du temps. Elles sont visualisées comme étant intactes après un mois de conservation à l'étuve à 45°C.

### Exemple 32 de l'invention :

Les 100 ml d'huile de paraffine fluide utilisés dans l'exemple 31-b) peuvent être remplacés avantageusement par une quantité comprise entre 100 et 500 ml d'huile de paraffine fluide.

### Exemple 33 de l'invention :

Les 100 ml d'huile de paraffine fluide utilisés dans l'exemple 31-b) peuvent être remplacés avantageusement par : 100 ml de myristate d'éthyle ou 100 ml de myristate d'isopropyle, ou 100 ml d'oléate d'éthyle, ou 100 ml d'acétate de vitamine E, ou 100 ml de palmitate de vitamine A ou 100 ml de benzoate de benzyle. Tout autre actif huileux ou huile végétale ou combinaison de ces dernières peuvent également être encapsulés de cette même façon.

### Exemple 34 de l'invention :

Il est possible de remplacer la phase a) de l'exemple 31 par la préparation suivante : 200 g de concentrat de protéines de féverole, dispersés dans 41 de tampon acétate pH 7,4. Après mélange et chauffage léger pendant 15 min à 35°C, la solution est centrifugée et le surnageant est utilisé pour la suite du procédé.

### Exemple 35 de l'invention :

### Préparation de nanosphères à paroi en protéines végétales

### a) Fabrication du mélange nécessaire à la fabrication des nanosphères

75 g de farine de lupin (farine ultra-fine de lupin blanc doux, (CANA) à 45 % de protéines) sont dispersés dans 1,5 1 de tampon acétate pH 7,4. Après agitation à température ambiante pendant 10 min, la solution est centrifugée et le surnageant est utilisé pour la suite du procédé.

### b) Préparation de l'agent réticulant

400 g de chlorure de téréphtaloyle sont broyés dans un mortier et sont rajoutés à un litre de vaseline visqueuse CODEX. L'ensemble est agité par agitation mécanique.

### c) Emulsification

61 d'huile de vaseline visqueuse CODEX (indice de viscosité : 250 centipoises) sont introduits dans une cuve sous agitation et 320 ml d'un tensioactif, (par exemple : le glycérol sorbitan hydroxyisostéarate ARLACEL 780, ICI) sont rajoutés à l'ensemble. L'ensemble est agité pendant quelques minutes. 1 kg de la solution telle que préparée en a) est alors introduit sous agitation, l'émulsifcation étant réalisée en quelques minutes à 20 000 rpm à l'aide d'un Ultra-Turax.

### d) Réticulation

La solution contenant l'agent réticulant préparée à l'étape b) est alors introduite dans l'émulsion, les particules solides présentes dans celle-ci sont alors rajoutées également et se dissoudront au cours du temps. Après 5 min d'agitation à 20 000 rpm à l'aide de l'Ultra-Turax, la solution est mise sous agitation mécanique à vitesse de rotation réduite pendant 18 h au moins à température ambiante. Les nanosphères sont séparées par centrifugation en discontinu et le surnageant est éliminé (4 000 rpm pendant 15 min).

### e) Lavage

Les nanosphères sont lavées par des lavages successifs réalisés à l'aide d'une phase organique miscible avec l'huile de vaseline. Citons pour l'exemple le DRAGOXAT® (DRAGOCO), le myristate d'isopropyl (STEARINERIE DUBOIS) et des triglycérides à chaîne moyenne (STEARINERIE DUBOIS). Au cours de chaque lavage, 100 ml de nanocapsules sont rajoutés à 500 ml de phase organique. L'ensemble est agité pendant quelques minutes puis centrifugé (4 000 rpm pendant 15 mn). Les nanosphères obtenues peuvent être mises en suspension, par exemple dans des gels de protéine ou de polysaccharides, dans une phase huileuse, ou dans un gel de polymères carboxyvinyliques (carbomer).

### Exemple 36 de l'invention :

### Préparation de nanosphères contenant un principe actif hydrosoluble ou insoluble

On procède comme décrit à l'exemple 35, si ce n'est qu'à la solution fabriquée en a), on peut rajouter de nombreux principes actifs, par exemple : du glucose, un acide aminé tels que la glutamine, la sérine, la glycine, la cystéine, les principes actifs tels que la caféine, la théophylline, les extraits de plante comme les extraits de gingko biloba, de centella asiatica, de marron d'inde.

### Exemple 37 de l'invention :

On procède comme décrit à l'exemple 36. Cependant la préparation de la phase telle que décrite en a) est modifiée de la façon suivante :
37-A) Remplacement du tampon acétate pH 7,4 par un tampon acétate pH 6,8.
37-B) Remplacement du tampon acétate pH 7,4 par un tampon acétate pH 5,9.
37-C) Remplacement du tampon acétate pH 7,4 par un tampon succinate pH 6.
37-D) Remplacement du tampon acétate pH 7,4 par un tampon citrate pH 6.
37-E) Remplacement de la farine de lupin par un isolat de protéines de pois.
37-F) Remplacement de la farine de lupin par un concentrat de protéines de féverole (à 50% de protéines : Concentrat 50® de GEMEF INDUSTRIE).

### Exemple 38 de l'invention :

### Emulsion eau-dans-huile à usage cosmétique ou pharmaceutique

### Utilisation des microparticules ou nanoparticules dans des formulations de type émulsion eau-dans-huile

| Formulation 38-A | | |
|---|---|---|
| A | Eau | qsp 100 |
| | Butylène Glycol | 2 |
| | Glycerine | 3 |
| | Sodium Dihydroxycetyl Phosphate, Isopropyl Hydroxycetyl Ether | 2 |
| | | |
| B | Glycol Stearate SE | 14 |
| | Triisononanoin | 5 |
| | Octyl Cocoate | 6 |
| | | |
| C | Butylene Glycol, Methylparaben, Ethylparaben, Propylparaben. | 2 |
| | pH ajusté à 5,5 | |
| | | |
| D | Produits de l'invention | 0,01 - 10 %. |

| Formulation 38-B | | |
|---|---|---|
| A | Eau | qsp 100 |
| | Butylene Glycol | 2 |
| | Glycerine | 3 |
| | Polyacrylamide, Isoparaffin. Laureth-7 | 2,8 |
| | | |
| B | Butylene Glycol Methylparaben. Ethylparaben. Propylparaben | 2 |
| | | |
| | Phenoxyethanol, Methylparaben, Propylparaben, Butylparaben. Ethylparaben | 0,5 |
| | | |
| | Butylene Glycol | 0,5 |
| | | |
| C | Produits de l'invention | 0,01 - 10 % |

| Formulation 38-C | | |
|---|---|---|
| A | Carbomer | 0,50 |
| | Propylene Glycol | 3,00 |
| | Glycerol | 5,00 |
| | Eau | qsp 100 |
| B | Octyl Cocoate | 5,00 |
| | Bisabolol | 0,30 |
| | Dimethicone | 0,30 |
| | | |
| C | Sodium Hydroxide | 1,60 |
| | | |
| D | Phenoxyethanol Methylparaben, Ethylparaben, Propylparaben, Butylparaben | 0,50 |
| | | |
| | Parfum | 0,3 |
| E | Produit de l'invention | 0,01 - 10 %. |

### Exemple 39 de l'invention :

### Emulsion huile-dans-eau à usage cosmétique ou pharmaceutique

### Utilisation des microparticules et des nanoparticules dans une formulation de type huile-dans-eau

| | | |
|---|---|---|
| A | PEG 30-dipolyhydroxystearate | 3 |
| | Capric Triglycerides | 3 |
| | Cetearyl Octanoate | 4 |
| | Dibutyl Adipate | 3 |
| | Grape Seed Oil | 1,5 |
| | Jojoba Oil | 1,5 |
| | Phenoxyethanol, Methylparaben, Ethylparaben, Propylparaben, Butylparaben | 0,5 |
| | | |
| B | Glycerine | 3 |
| | Butylene Glycol | 3 |
| | Magnesium Sulfate | 0,5 |
| | EDTA | 0,05 |
| | Eau | qsp 100 |
| | | |
| C | Cyclomethicone | 1 |
| | Dimethicone | 1 |
| | | |
| D | Parfum | 0,3 |
| | | |
| E | Produit de l'invention | 0,01 - 10% |

### Exemple 40 de l'invention :

### Composition cosmétique

### Utilisation des microparticules et des nanoparticules dans une formulation de type shampooing ou gel douche

| | | |
|---|---|---|
| A | Xanthan Gum | 0,8 |
| | Eau | qsp 100 |
| | | |
| B | Phenoxyethanol Methylparaben, Ethylparaben, Propylparaben, Butylparaben | 0,5 |
| | | |
| | Butylene Glycol Methylparaben, Ethylparaben, Propylparaben | 0,5 |
| | | |
| C | Citric acid | 0,8 |
| | | |
| D | Sodium Laureth Sulfate | 40,0 |
| | | |
| E | Produit de l'invention | 0,01 - 10 % |

### Exemple 41 de l'invention :

### Composition cosmétique

### Utilisation des microparticules et des nanoparticules dans une formulation de type rouge à lèvres et autres produits anhydres

| | | |
|---|---|---|
| A | Mineral Wax | 17,0 |
| | Isostearyl Isostéarate | 31,5 |
| | Propylene Glycol Dipelargonate | 2,6 |
| | Propylene Glycol Isostearate | 1,7 |
| | PEG 8 Beeswax | 3,0 |
| | Hydrogenated Palm Kernel Oil Glycerides, Hydrogenated Palm Glyceride | 3,4 |
| | Lanoline Oil | 3,4 |
| | Sesame Oil | 1,7 |
| | Tribehenin | 1,7 |
| | Cetyl Lactate | 1,7 |
| | Minéral Oil, Lanolin Alcohol | 3,0 |
| | | |
| B | Castor Oil | qsp 100 |
| | | |
| | Titanium Dioxide | 3,9 |
| | CI 15850:1 | 0,616 |
| | CI 45410:1 | 0,256 |
| | CI 19140:1 | 0,048 |
| | CI 77491 | 2,048 |
| | | |
| C | Produit de l'invention | 0,01 - 5 |

### Exemple 42 de l'invention :

### Composition cosmétique

### Utilisation des microparticules et des nanoparticules dans une formulation de gels aqueux (contours de l'oeil, amincissants, etc.)

| | |
|---|---|
| Eau | qsp 100 |
| | |
| Carbomer | 0,5 |
| | |
| Butylene Glycol | 15 |
| | |
| Phenoxyethanol, Methylparaben, Ethylparaben, Propylparaben, Butylparaben | 0,5 |
| Produit de l'invention | 0,01-10 |

### Exemple 43 de l'invention :

### Etudes toxicologiques réalisées sur les produits de l'exemple 25 de l'invention

### a) Toxicité orale

Les tests ont été effectués en suivant le protocole en accord avec la ligne directrice de l'OCDE concernant l'étude de la toxicité orale aiguë (n°401 du 24 février 1987) à des doses maximales de 5 g/kg de poids corporel et n'ont provoqué aucune lésion macroscopique pouvant être rapportée à un effet toxique du produit.

Les produits de l'invention (microcapsules de soja) tels qu'obtenus à l'exemple 25 sont utilisés oralement en dose inférieure à 5 g/kg et présentent donc une toxicité nulle.

### b) Irritation oculaire

Les tests ont été effectués selon la méthode officielle définie par l'arrêté du 3 mai 1990 (Journal Officiel de la République Française du 14 novembre 1990) avec le produit de l'invention (microcapsules de soja) et n'ont provoqué aucune lésion de l'iris ou de la cornée.

Les produits de l'invention (microcapsules de soja) instillés purs sont apparus non irritants et la tolérance oculaire peut être considérée comme très bonne.

### c) Irritation cutanée

Les tests ont été effectués selon la méthode officielle définie par l'arrêté du 1^{er} février 1982 (Journal Officiel de la République Française du 21 février 1982) avec le produit de l'invention (microcapsules de soja) et n'ont provoqué aucun phénomène irritatif.

Les produits de l'invention (microcapsules de soja) instillés purs sont apparus non irritants et la tolérance cutanée peut être considérée comme excellente.

### d) Recherche du pouvoir sensibilisant

Des tests de maximisation ont été réalisés selon un protocole adapté de la méthode décrite par MAGNUSSON et KLIGMAN (J. INVEST. DERM. 1969, 52, 268-276).

Les produits de l'invention (microcapsules de soja) n'ont provoqué aucune réaction macroscopique significative d'une réaction de sensibilisation. Ils peuvent donc être considérés comme hypoallergéniques (classe I).

## Revendications

1. Particules, **caractérisées en ce qu'**elles comprennent au moins en surface, une paroi formée de protéines végétales réticulées, en particulier au moyen d'une réticulation interfaciale entre les protéines végétales et un agent réticulant polyfonctionnel acylant, comprenant au moins deux groupes acylants, réalisant des liaisons covalentes entre les fonctions acylables des protéines et les groupements acyles de l'agent réticulant polyfonctionnel acylant.

2. Particules selon la revendication 1, **caractérisées en ce que** les protéines végétales précitées sont extraites notamment de légumineuses, en particulier des plantes suivantes : lupin (genre Lupinus), soja (genre Glycine), pois (genre Pisum), pois chiche (Cicer), de la luzerne (Medicago), féverole (Vicia), lentille (Lens), haricot (Phaseolus), du colza (Brassica), ou du tournesol (Helianthus), ou encore de céréales comme le blé, le maïs, l'orge, le malt, l'avoine.

3. Particules selon la revendication 1 ou 2, **caractérisées en ce que** les protéines végétales précitées sont utilisées sous forme de préparations pulvérulentes telles que des farines, des concentrats, des isolats, ou de préparations liquides telles que des laits de soja.

4. Particules selon la revendication 3, **caractérisées en ce que** les protéines végétales sont dissoutes, à une concentration en poids comprise entre 0,5 et 5 % dans une solution aqueuse de pH compris entre environ 4,5 et environ 8.

5. Particules selon la revendication 4 **caractérisées en ce que** la solution aqueuse de protéine végétale de pH compris entre environ 4,5 et environ 8 renferme au moins un sel d'acide carboxylique à une concentration en poids comprise entre 0,1 et 20 %.

6. Particules selon la revendication 5 **caractérisée en ce que** le sel d'acide carboxylique est un sel alcalin ou alcalino-terreux d'un acide carboxylique choisi parmi le groupe consistant de l'acide acétique, oxalique, malonique, succinique, glutarique, diméthylglutarique, adipique, famarique, maléique, tartrique, malique, citrique, lactique, et l'acide salicylique.

7. Particules selon l'une des revendications précédentes, **caractérisées en ce qu'**elles contiennent une substance insoluble lipophile, en particulier choisie parmi le groupe consistant des sels insolubles d'acides gras et de métaux bivalents comme le stéarate de calcium ou de magnésium, des oxydes métalliques tels que l'oxyde de titane, l'oxyde de zinc, ou du talc, ou des substances colorées insolubles sous forme de pigments ou sous la forme de laques de calcium, d'aluminium, de baryum ou de zirconium de divers colorants.

8. Particules selon la revendication 7, **caractérisées en ce que** les sels insolubles d'acides gras précités sont choisis parmi le groupe consistant des sels de calcium, magnésium, strontium, baryum, d'acides carboxyliques à nombre de carbones égal ou supérieur à 12, tels que les acides laurique, myristique, palmitique, stéarique, oléique, linoléique, et les laques précitées sont choisies parmi le groupe consistant de l'indigocarmine aluminium lake (de couleur bleue), le Ponceau 4 R aluminium lake (rouge), le Sunset yellow FCF aluminium lake (orange).

9. Particules selon l'une des revendications précédentes, **caractérisées en ce que** l'agent réticulant polyfonctionnel acylant précité est constitué par un dihalogénure d'acide ou un dianhydride d'acide.

10. Particules selon la revendication 9, **caractérisées en ce que** le dihalogénure d'acide est choisi parmi le dihalogénure de phtaloyle, de téréphtaloyle, de sébacoyle, de glutaryle, d'adipoyle ou de succinyle.

11. Particules selon l'une des revendications précédentes, **caractérisées en ce qu'**elles contiennent un principe actif cosmétique, pharmaceutique ou alimentaire tel que huile végétale, minérale ou synthétique, des dérivés de vitamine A et de vitamine E, des principes actifs hydrophiles tels que des extraits végétaux, l'acide ascorbique ou vitamine C, PMG, glucose, des pigments organiques et des pigments minéraux.

12. Particules selon l'une des revendications précédentes, **caractérisées en ce qu'**elles ont une taille comprise entre environ 10 nanomètres et environ 3 millimètres.

13. Utilisation des particules selon l'une quelconque des revendications précédentes pour la fabrication d'une composition cosmétique, pharmaceutique et notamment dermatologique, ou alimentaire.

14. Composition cosmétique, pharmaceutique, notamment dermatologique ou alimentaire, contenant des particules selon l'une quelconque des revendications 1 à 12.

15. Procédé de fabrication de particules de protéines végétales réticulées, **caractérisé en ce qu'**il comprend :
a) la formation d'une solution aqueuse de pH compris entre environ 4,5 et environ 8 contenant en solution au moins une protéine végétale et au moins un sel d'acide carboxylique ;
b) la formation d'une phase huileuse ;
c) la formation d'une émulsion par mélange sous agitation de la phase aqueuse et de la phase huileuse ci-dessus ;
d) la réticulation interfaciale de ladite protéine végétale à l'aide d'un agent réticulant polyfonctionnel acylant à au moins deux groupes acylants pendant une période de temps suffisante pour obtenir des particules comprenant au moins en surface des parois formées de protéines végétales réticulées par ledit agent réticulant ; et
e) si désiré, la séparation des particules ainsi préparées.

16. Procédé selon la revendication 15, **caractérisé en ce que** la concentration en poids de la protéine végétale dans la phase aqueuse est comprise entre environ 0,5 et environ 5 %.

17. Procédé selon la revendication 15 ou 16, **caractérisé en ce que** la solution aqueuse précitée à pH compris entre environ 4,5 et environ 8 renferme une quantité d'un sel d'acide carboxylique comprise généralement entre environ 0,1 et 20 % en poids.

18. Procédé selon l'une des revendications 15 à 17, **caractérisé en ce que** l'agent réticulant polyfonctionnel précité est choisi parmi un dihalogénure d'acide ou un dianhydride d'acide.

19. Procédé selon l'une des revendications 15 à 18, **caractérisé en ce que** le rapport du poids de l'agent réticulant polyfonctionnel précité au poids de protéine végétale utilisée est compris généralement entre 0,03 et 70 parties en poids d'agent réticulant pour une partie en poids de protéines.

20. Procédé selon l'une des revendications 15 à 19, **caractérisé en ce qu'**on utilise pour former la phase huileuse soit un solvant organique qui peut être facilement éliminé tel que le cyclohexane ou un mélange chloroforme/cyclohexane en particulier à un rapport 1:4 v/v, soit de préférence une huile biocompatible, en particulier une huile végétale biocompatible, telle qu'une huile de coco, ou le cocoate d'éthyle-2-hexyle, ou une huile de paraffine.

21. Procédé selon l'une des revendications 15 à 20, **caractérisé en ce qu'**on réalise un procédé en émulsion de type eau-dans-huile.

22. Procédé selon l'une des revendications 15 à 20, **caractérisé en ce qu'**on réalise un procédé en émulsion de type huile-dans-eau.

23. Procédé selon l'une des revendications 15 à 22, **caractérisé en ce qu'**on utilise un agent tensioactif pour faciliter ou stabiliser l'émulsion, de préférence un tensioactif de type sorbitan, tel que le sorbitan trioléate.

24. Procédé selon l'une des revendications 15 à 23, **caractérisé en ce que**, pour augmenter la taille des particules, on ajoute dans la phase aqueuse ou la phase hydrophobe ou à l'une et l'autre phase, une substance insoluble lipophile choisie de préférence parmi le groupe des sels insolubles d'acides gras et de métaux bivalents, du talc, des oxydes métalliques, ou des substances colorées insolubles sous forme de pigment ou sous la forme de laque.

25. Procédé selon la revendication 24, **caractérisé en ce que** les sels insolubles d'acides gras et de métaux bivalents précités sont choisis parmi le groupe consistant des sels de calcium, magnésium, strontium, baryum, d'acides carboxyliques à nombre de carbones égal ou supérieur à 12, tels que les acides laurique, myristique, palmitique, stéarique, oléique, linoléique, les oxydes métalliques précités sont choisis parmi le groupe consistant de l'oxyde de titane, l'oxyde de zinc, les substances colorées insolubles sont choisies parmi le groupe des pigments organiques tels que le DC Red 30 ou des laques choisies parmi des laques de calcium, d'aluminium, de baryum ou de zirconium de colorants, telles que l'indigocarmine aluminium lake de couleur bleue, le Ponceau 4R aluminium lake, rouge, ou le Sunset yellow FCF aluminium lake, orange.

26. Procédé selon la revendication 24 ou 25, **caractérisé en ce que** la substance insoluble lipophile précitée est incorporée en une quantité comprise entre 0,01 % et 2 % en poids de la phase concernée.

27. Procédé selon l'une des revendications 15 à 26, **caractérisé en ce que** la réaction de réticulation interfaciale est réalisée à une température comprise entre 4 et 80°C, de préférence entre 15 et 30°C, et à pression atmosphérique.

28. Procédé selon l'une des revendications 15 à 27 **caractérisée en ce que** le sel d'acide carboxylique est un sel alcalin ou alcalino-terreux d'un acide carboxylique choisi parmi le groupe consistant de l'acide acétique, oxalique, malonique, succinique, glutarique, diméthylglutarique, adipique, fumarique, maléique, tartrique, malique, citrique, lactique, et l'acide salicylique.

## Patentansprüche

1. Partikel, **dadurch gekennzeichnet, dass** sie zumindest auf der Oberfläche eine Wand umfassen, die aus pflanzlichen Proteinen gebildet ist, die insbesondere mittels einer Grenzflächenvernetzung zwischen den pflanzlichen Proteinen und einem polyfunktionellen Acylierungsvernetzungsmittel vernetzt sind, welches mindestens zwei Acylierungsgruppen umfasst, die kovalente Bindungen zwischen den acylierbaren Funktionen der Proteine und den Acyl-Gruppierungen des polyfunktionellen Acylierungsvernetzungsmittels ergeben.

2. Partikel gemäß Anspruch 1, **dadurch gekennzeichnet, dass** die vgenannten pflanzlichen Proteine besonders aus Leguminosen extrahiert sind, insbesondere aus den folgenden Pflanzen: Lupinen (Genus Lupinus), Soja (Genus Glycin), Erbse (Genus Pisum), Kichererbse (Cicer), Luzerne (Medicago), Saubohne (Vicia), Linse (Lens), Bohne (Phaseolus), Raps (Brassica) oder Sonnenblume (Helianthus) oder auch aus Getreidesorten wie Weizen, Mais, Gerste, Malz und Hafer.

3. Partikel gemäß Anspruch 1 oder 2, **dadurch gekennzeichnet, dass** die genannten pflanzlichen Proteine in Form von pulverförmigen Zubereitungen wie Mehlen, Konzentraten und Isolaten oder in Form von flüssigen Zubereitungen wie aus Soja-Milch verwendet werden.

4. Partikel gemäß Anspruch 3, **dadurch gekennzeichnet, dass** die pflanzlichen Proteine mit einer Konzentration von 0,5 bis 5 Gew.% in einer wässrigen Lösung mit einem pH-Wert von ca. 4,5 bis ca. 8 gelöst sind.

5. Partikel gemäß Anspruch 4, **dadurch gekennzeichnet, dass** die wässrige Lösung aus pflanzlichem Protein mit dem pH-Wert von ca. 4,5 bis 8 mindestens ein Salz einer Carbonsäure mit einer Konzentration von 0,1 bis 20 Gew.% einschließt.

6. Partikel gemäß Anspruch 5, **dadurch gekennzeichnet, dass** das Salz der Carbonsäure ein Alkali- oder Erdalkalisalz einer Carbonsäure ist, ausgewählt aus der Gruppe, bestehend aus Essig-, Oxal-, Malon-, Bernstein-, Glutar-, Dimethylglutar-, Adipin-, Fumar-, Malein-, Wein-, Apfel-, Zitronen-, Milch- und aus Salicylsäure.

7. Partikel gemäß einem der vorhergehenden Ansprüche, **dadurch gekennzeichnet, dass** sie eine unlösliche lipophile Substanz enthalten, insbesondere ausgewählt aus der Gruppe, bestehend aus unlöslichen Salzen von Fettsäuren und zweiwertigen Metallen, wie Calcium- oder Magnesiumstearat, Metalloxiden wie Titan- oder Zinkoxid oder Talkum oder aus gefärbten unlöslichen Substanzen in Form von Pigmenten oder in Form von Lacken aus Calcium, Aluminium, Barium oder Zirkon mit verschiedenen Farbstoffen.

8. Partikel gemäß Anspruch 7, **dadurch gekennzeichnet, dass** die genannten Salze unlöslicher Fettsäuren aus der Gruppe ausgewählt sind, bestehend aus Salzen von Calcium, Magnesium, Strontium und Barium von Carbonsäuren mit einer Kohlenstoffzahl gleich oder mehr als 12, wie von Laurin-, Myristin-, Palmitin-, Stearin-, Öl- und Linolsäure, und die vorgenannten Lacke aus der Gruppe, bestehend aus Indigocarmin-, Aluminium-Lake (blauer Farbe), Ponceau 4 R-Aluminuim-Lake (Rot) und aus Sunset-Gelb-FCF-Aluminium-Lake (Orange), ausgewählt sind.

9. Partikel gemäß einem der vorhergehenden Ansprüche, **dadurch gekennzeichnet, dass** das genannte polyfunktionelle Acylierungsvernetzungsmittel aus einem Säuredihalogenid oder einem Säuredianhydrid dargestellt ist.

10. Partikel gemäß Anspruch 9, **dadurch gekennzeichnet, dass** das Säuredihalogenid aus Phthaloyl-, Terephthaloyl-, Sebacoyl-, Glutaryl-, Adipoyl- oder aus Bernsteinsäuredihalogenid ausgewählt ist.

11. Partikel gemäß einem der vorhergehenden Ansprüche, **dadurch gekennzeichnet, dass** sie ein kosmetisches, pharmazeutisches oder Nahrungsmittel-bezogenes Wirkprinzip wie pflanzliches, mineralisches oder synthetisches Öl, Derivate von Vitamin A und Vitamin E, hydrophile Wirkprinzipe wie Pflanzenextrakte, Ascorbinsäure oder Vitamin C, PMG, Glucose, organische Pigmente und mineralische Pigmente enthalten.

12. Partikel gemäß einem der vorhergehenden Ansprüche, **dadurch gekennzeichnet, dass** sie eine Größe von ca. 10 nanometer bis ca. 3 millimeter aufweisen.

13. Verwendung der Partikel gemäß einem der vorhergehenden Ansprüche zur Herstellung einer kosmetischen, pharmazeutischen und insbesondere dermatologischen oder einer Nahrungsmittel-bezogenen Zusammensetzung.

14. Kosmetische, pharmazeutische, insbesondere dermatologische oder Nahrungsmittel-bezogene Zusammensetzung, enthaltend Partikel gemäß einem der Ansprüche 1 bis 12.

15. Verfahren zur Herstellung von Partikeln aus vernetzten pflanzlichen Proteinen, **dadurch gekennzeichnet, dass** es umfasst:
a) die Bildung einer wässrigen Lösung mit einem pH-Wert von ca. 4,5 bis ca. 8, die mindestens ein pflanzliches Protein und mindestens ein Carbonsäuresalz enthält;
b) die Bildung einer Öl-Phase;
c) die Bildung einer Emulsion durch Mischen unter Rühren der wässrigen Phase und der obigen Öl-Phase;
d) die Grenzflächenvernetzung des genannten pflanzlichen Proteins mittels eines polyfunktionellen Acylierungsvernetzungsmittels mit mindestens zwei Acylierungsgruppen während einer Zeitdauer, die ausreicht, um Partikel zu erhalten, die zumindest an der Oberfläche Wände umfassen, die aus pflanzlichen Proteinen gebildet sind, die mit dem genannten Vernetzungsmittel vernetzt sind; und
e) gegebenenfalls die Abtrennung der so hergestellten Partikel.

16. Verfahren gemäß Anspruch 15, **dadurch gekennzeichnet, dass** die Konzentration des pflanzlichen Proteins in der wässrigen Phase ca. 0,5 bis ca. 5 Gew.% beträgt.

17. Verfahren gemäß Anspruch 15 oder 16, **dadurch gekennzeichnet, dass** die genannte wässrige Lösung mit einem pH-Wert von ca. 4,5 bis ca. 8 eine Menge eines Carbonsäuresalzes aus im allgemeinen ca. 0,1 bis 20 Gew.% einschließt.

18. Verfahren gemäß einem der Ansprüche 15 bis 17, **dadurch gekennzeichnet, dass** das genannte polyfunktionelle Vernetzungsmittel aus einem Säuredihalogenid oder einem Säuredianhydrid ausgewählt ist.

19. Verfahren gemäß einem der Ansprüche 15 bis 18, **dadurch gekennzeichnet, dass** das Gewichtsverhältnis des genannten polyfunktionellen Vernetzungsmittels zum Gewicht von eingesetztem pflanzlichen Protein im allgemeinen 0,03 bis 70 Gew.Teile Vernetzungsmittel pro 1 Gew.Teil Proteine beträgt.

20. Verfahren gemäß einem der Ansprüche 15 bis 19, **dadurch gekennzeichnet, dass** man zur Bildung der Öl-Phase entweder ein organisches Lösungsmittel, das leicht entfernt werden kann, wie Cyclohexan oder eine Mischung aus Chloroform/Cyclohexan mit insbesondere einem Verhältnis von 1:4 V/V, oder vorzugsweise ein biokompatibles Öl, insbesondere ein biokompatibles pflanzliches Öl wie Kokosöl oder Ethyl-2-Hexylcocoat, oder ein Paraffinöl verwendet.

21. Verfahren gemäß einem der Ansprüche 15 bis 20, **dadurch gekennzeichnet, dass** man ein Verfahren in Emulsion vom Wasser-in-Öl-Typ durchführt.

22. Verfahren gemäß einem der Ansprüche 15 bis 20, **dadurch gekennzeichnet, dass** man ein Verfahren in Emulsion vom Öl-in-Wasser-Typ durchführt.

23. Verfahren gemäß einem der Ansprüche 15 bis 22, **dadurch gekennzeichnet, dass** man ein oberflächenaktives Mittel zur Erleichterung oder Stabilisierung der Emulsion, vorzugsweise ein oberflächenaktives Mittel vom Sorbitan-Typ, wie Sorbitantrioleat, verwendet.

24. Verfahren gemäß einem der Ansprüche 15 bis 23, **dadurch gekennzeichnet, dass** man zur Vergrößerung der Partikelgröße in die wässrige oder hydrophobe Phase oder in die eine oder in die andere Phase eine unlösliche lipophile Substanz gibt, die vorzugsweise aus der Gruppe aus unlöslichen Salzen von Fettsäuren und zweiwertigen Metallen, aus Talkum, Metalloxiden oder aus unlöslichen gefärbten Substanzen in Form eines Pigments oder in Form von Lack ausgewählt ist.

25. Verfahren gemäß Anspruch 24, **dadurch gekennzeichnet, dass** die genannten unlöslichen Salze aus Fettsäuren und 2-wertigen Metallen, aus der Gruppe, bestehend aus Salzen von Calcium, Magnesium, Strontium oder Barium und von Fettsäuren mit einer Kohlenstoffzahl gleich oder höher als 12, wie Laurin-, Myristin-, Palmitin-, Stearin-, Öl- oder Linolsäure, die genannten Metalloxide aus der Gruppe, bestehend aus Titan- oder Zinkoxid, die gefärbten unlöslichen Substanzen aus der Gruppe aus organischen Pigmenten wie dem DC Red 30 oder aus Lacken aus Lacken von Calcium, Aluminium, Barium oder von Zirkon mit Farbstoffen, wie aus Indigocarmin-Aluminuim-Lake blauer Farbe, rotem Ponceau 4R-Aluminium-Lake oder aus orangefarbenem Sunset-Gelb-FCF-Aluminium-Lake, ausgewählt ist.

26. Verfahren gemäß Anspruch 24 oder 25, **dadurch gekennzeichnet, dass** die genannte unlösliche lipophile Substanz in einer Menge von 0,01 bis 2 Gew.% der betreffenden Phase eingebracht wird.

27. Verfahren gemäß einem der Ansprüche 15 bis 26, **dadurch gekennzeichnet, dass** die Grenzflächenvernetzungsreaktion bei einer Temperatur von 4 bis 80 und vorzugsweise von 15 bis 30°C bei atmosphärischem Druck durchgeführt wird.

28. Verfahren gemäß einem der Ansprüche 15 bis 27, **dadurch gekennzeichnet, dass** das Carbonsäuresalz ein Alkalioder Erdalkalisalz einer Carbonsäure ist, die aus der Gruppe, bestehend aus Essig-, Oxal-, Malon-, Bernstein-, Glutar-, Dimethylglutar-, Adipin-, Fumar-, Malein-, Wein-, Apfel-, Zitronen-, Milch- und aus Salicylsäure, ausgewählt ist.

## Claims

1. Particles, **characterised in that** they comprise at least on the surface, a wall formed of plant proteins crosslinked, in particular by means of interfacial crosslinking between the plant proteins and an acylating polyfunctional crosslinking agent comprising at least two acylating groups, covalent bonds being formed between the acylatable functions of the proteins and the acyl groups of the acylating polyfunctional crosslinking agent.

2. Particles according to claim 1, **characterised in that** the above-mentioned plant proteins are extracted especially from leguminous plants, particularly from the following plants: lupin (genus Lupinus), soya (genus Glycine), pea (genus Pisum), chick pea (Cicer), lucerne (Medicago), horse bean (Vicia), lentil (Lens), bean (Phaseolus), colza (Brassica) or sunflower (Helianthus), or else from cereals such as wheat, maize, barley, malt and oats.

3. Particles according to claim 1 or 2, **characterised in that** the above-mentioned plant proteins are used in the form of pulverulent preparations such as flours, concentrates or isolates, or liquid preparations such as soya milks.

4. Particles according to claim 3, **characterised in that** the plant proteins are dissolved at a concentration of between 0.5 and 5% by weight in an aqueous solution with a pH of between about 4.5 and about 8.

5. Particles according to claim 4, **characterised in that** the aqueous solution of plant protein with a pH of between about 4.5 and about 8 contains at least one carboxylic acid salt at a concentration of between 0.1 and 20% by weight.

6. Particles according to claim 5, **characterised in that** the carboxylic acid salt is an alkali metal or alkaline earth metal salt of a carboxylic acid selected from the group comprising acetic, oxalic, malonic, succinic, glutaric, dimethylglutaric, adipic, fumaric, maleic, tartaric, malic, citric, lactic and salicylic acids.

7. Particles according to one of the preceding claims, **characterised in that** they contain an insoluble lipophilic substance selected in particular from the group comprising insoluble salts of fatty acids and divalent metals, such as calcium or magnesium stearate, metal oxides such as titanium oxide or zinc oxide, talcum, or insoluble coloured substances in the form of pigments or in the form of calcium, aluminum, barium or zirconium lakes of various colourants.

8. Particles according to claim 7, **characterised in that** the above-mentioned insoluble fatty acid salts are selected from the group consisting of the calcium, magnesium, strontium and barium salts of carboxylic acids with a number of carbons equal to or greater than 12, such as lauric, myristic, palmitic, stearic, oleic and linoleic acids, and the above-mentioned lakes are selected from the group comprising indigo carmine aluminum lake (with a blue colour), Ponceau 4R aluminum lake (red) and Sunset yellow FCF aluminum lake (orange).

9. Particles according to one of the preceding claims, **characterised in that** the above-mentioned acylating polyfunctional crosslinking agent consists of an acid dihalide or an acid dianhydride.

10. Particles according to claim 9, **characterised in that** the acid dihalide is selected from phthaloyl, terephthaloyl, sebacoyl, glutaryl, adipoyl or succinyl dihalides.

11. Particles according to one of the preceding claims, **characterised in that** they contain a cosmetic, pharmaceutical or food active principle such as vegetable, mineral or synthetic oil, vitamin A and vitamin E derivatives and hydrophilic active principles such as plant extracts, ascorbic acid, vitamin C, PMG, glucose, organic pigments and inorganic pigments.

12. Particles according to one of the preceding claims, **characterised in that** they have a size of between about 10 nanometers and about 3 millimeters.

13. Use of the particles according to any one of the preceding claims for the manufacture of a cosmetic or pharmaceutical composition, especially a dermatological composition, or a food composition.

14. A cosmetic or pharmaceutical composition, especially a dermatological composition, or a food composition containing particles according to any one of claims 1 to 12.

15. A process for the manufacture of particles of crosslinked plant proteins, **characterised in that** it comprises:
a) the formation of an aqueous solution with a pH of between about 4.5 and about 8 which contains, in solution, at least one plant protein and at least one carboxylic acid salt;
b) the formation of an oily phase;
c) the formation of an emulsion by mixing the above aqueous phase and oily phase, with agitation;
d) the interfacial crosslinking of said plant protein with an acylating polyfunctional crosslinking agent having at least two acylating groups, for a sufficient period of time to obtain particles comprising, at least on the surface, walls formed of plant proteins crosslinked by said crosslinking agent; and
e) if desired, the separation of the particles prepared in this way.

16. A process according to claim 15, **characterised in that** the concentration of the plant protein in the aqueous phase is between about 0.5 and about 5% by weight.

17. A process according to claim 15 or 16, **characterised in that** the above-mentioned aqueous solution with a pH of between about 4.5 and about 8 contains an amount of carboxylic acid salt which is generally between about 0.1 and 20% by weight.

18. A process according to one of claims 15 to 17, **characterised in that** the above-mentioned polyfunctional crosslinking agent is selected from an acid dihalide or an acid dianhydride.

19. A process according to one of claims 15 to 18, **characterised in that** the ratio of the weight of the above-mentioned polyfunctional crosslinking agent to the weight of plant protein used is generally between 0.03 and 70 parts by weight of crosslinking agent to one part by weight of proteins.

20. A process according to one of claims 15 to 19, **characterised in that** the oily phase is formed using either an organic solvent which can easily be removed, such as cyclohexane or a chloroform/cyclohexane mixture, particularly in the ratio 1:4 v/v, or, preferably, a biocompatible oil, particularly a biocompatible vegetable oil such as a coconut oil, 2-ethylhexyl cocoate or a paraffin oil.

21. A process according to one of claims 15 to 20, **characterised in that** a water-in-oil type emulsion process is carried out.

22. A process according to one of claims 15 to 20, **characterised in that** a oil-in-water type emulsion process is carried out.

23. A process according to one of claims 15 to 22, **characterised in that** a surfactant, preferably a surfactant of the sorbitan type, such as sorbitan trioleate, is used to facilitate or stabilize the emulsion.

24. A process according to one of claims 15 to 23, **characterised in that**, to increase the particle size, an insoluble lipophilic substance is added to the aqueous phase, the hydrophobic phase or both phases, said substance preferably being selected from the group comprising insoluble salts of fatty acids and divalent metals, talcum, metal oxides, or insoluble coloured substances in the form of a pigment or in the form of a lake.

25. A process according to claim 24, **characterised in that** the above-mentioned insoluble salts of fatty acids and divalent metals are selected from the group comprising the calcium, magnesium, strontium and barium salts of carboxylic acids with a number of carbons equal to or greater than 12, such as lauric, myristic, palmitic, stearic, oleic and linoleic acids, the above-mentioned metal oxides are selected from the group comprising titanium oxide and zinc oxide, and the insoluble coloured substances are selected from the group comprising organic pigments such as DC Red 30, or lakes selected from the calcium, aluminum, barium or zirconium lakes of colorants, such as indigo carmine aluminum lake, which is blue, Ponceau 4R aluminum lake, which is red, or Sunset yellow FCF aluminum lake, which is orange.

26. A process according to claim 24 or 25, **characterised in that** the above-mentioned insoluble lipophilic substance is incorporated in an amount of between 0.01% and 2% by weight of the phase in question.

27. A process according to one of claims 15 to 26, **characterised in that** the interfacial crosslinking reaction is carried out at a temperature of between 4 and 80°C, preferably of between 15 and 30°C, and at atmospheric pressure.

28. A process according to one of claims 15 to 27, **characterised in that** the carboxylic acid salt is an alkali metal or alkaline earth metal salt of a carboxylic acid selected from the group comprising acetic, oxalic, malonic, succinic, glutaric, dimethylglutaric, adipic, fumaric, maleic, tartaric, malic, citric, lactic and salicylic acids.
